# EUROPEAN PATENT SPECIFICATION

(11) **EP 2 802 349 B1**
(45) Date of publication and mention of the grant of the patent: **06.12.2017**
(21) Application number: 13736349.5
(22) Date of filing: 10.01.2013
(51) Int. Cl.: A61K 39/12, C12N 7/01, A61P 31/20

(54) **IMMUNOGENIC HPV L2-CONTAINING VLPS AND RELATED COMPOSITIONS AND METHODS**
IMMUNOGENE HPV L2-HALTIGE VLPS UND VERWANDTE ZUSAMMENSETZUNGEN UND VERFAHREN
VLP IMMUNOGÈNES CONTENANT LE HPV L2 ET COMPOSITIONS ET PROCÉDÉS ASSOCIÉS

(30) Priority: 12.01.2012 US 201261585839 P
(43) Date of publication of application: 19.11.2014
(73) Proprietor: STC.UNM, Albuquerque, NM 87106 (US)
(72) Inventor: CHACKERIAN, Bryce, Albuquerque, NM 87106 (US); PEABODY, David, Albuquerque, NM 87109 (US); TUMBAN, Ebenezer, Albuquerque, NM 87123 (US)
(74) Representative: Dall'Olio, Christian
(86) International application number: PCT/US2013/020960
(87) International publication number: WO 2013/106525

(56) References cited:
- WO-A2-2011/100234
- US-A1- 2002 136 736
- US-A1- 2003 175 290
- US-A1- 2009 028 886
- US-B1- 6 174 532
- TUMBAN EBENEZER ET AL: "A pan-HPV vaccine based on bacteriophage PP7 VLPs displaying broadly cross-neutralizing epitopes from the HPV minor capsid protein, L2.", PLOS ONE 2011, vol. 6, no. 8, E23310, 2011, pages 1-11, XP002741973, ISSN: 1932-6203
- PEABODY DAVID S ET AL: "Immunogenic display of diverse peptides on virus-like particles of RNA phage MS2.", JOURNAL OF MOLECULAR BIOLOGY 27 JUN 2008, vol. 380, no. 1, 27 June 2008 (2008-06-27) , pages 252-263, XP002741974, ISSN: 1089-8638
- TISSOT ALAIN C ET AL: "Versatile virus-like particle carrier for epitope based vaccines.", PLOS ONE 2010, vol. 5, no. 3, E9809, 2010, pages 1-8, XP002741975, ISSN: 1932-6203
- CALDEIRA J D C ET AL: "Immunogenic display of diverse peptides, including a broadly cross-type neutralizing human papillomavirus L2 epitope, on virus-like particles of the RNA bacteriophage PP7", VACCINE, ELSEVIER LTD, GB, vol. 28, no. 27, 17 June 2010 (2010-06-17) , pages 4384-4393, XP027064058, ISSN: 0264-410X, DOI: 10.1016/J.VACCINE.2010.04.049 [retrieved on 2010-05-28]
- TUMBAN EBENEZER ET AL: "VLPs displaying a single L2 epitope induce broadly cross-neutralizing antibodies against human papillomavirus.", PLOS ONE 2012, vol. 7, no. 11, E49751, 2012, pages 1-11, XP002741976, ISSN: 1932-6203

## Description

### RELATED APPLICATIONS AND GOVERNMENT INTEREST

This application claims the benefit of priority of United States provisional application serial number US61/585,839, filed January 12,2012, entitled "Immunogenic HPV L2-Containing VLPs and Related Compositions, Constructs and Therapeutic.

### FIELD OF THE INVENTION

The invention provides immunogenic HPV L2-containing viral-like particles (VLPs). Related compositions (e.g. vaccines) are also provided. The VLPs are comprised of a coat polypeptide of the bacteriophages MS2, wherein the coat protein is modified by insertion of peptide antigen corresponding to amino acids 17-31 from HPV L2, and wherein the HPV L2 peptide is displayed on the VLP. Specifically, VLPs of the invention display L2 said L2 peptide at the N-terminus of the bacteriophage coat protein. Surprisingly, these L2-displaying VLPs induce more broadly neutralizing antibody responses than when the same peptide is displayed in the AB-loop.

Immunogenic VLPs and related compositions of the invention induce high titer antibody responses against HPV L2 and protect against HPV pseudovirus challenge *in vivo.*

### BACKGROUND OF THE INVENTION

The growth of recombinant DNA technology in recent years has led to the introduction of vaccines in which an immunogenic protein has been identified, cloned and expressed in a suitable host to obtain sufficient quantities of protein to allow effective protective immunization in both animals and humans. Many of the most effective vaccines are based on the potent ability of virion surfaces to elicit neutralizing antibodies. These include licensed killed or attenuated virus vaccines, such as polio, influenza and rabies, which effectively induce protective antibody responses. More recently, subunit vaccines based upon self-assemblages of the structural proteins of human papillomavirus (HPV) and hepatitis B virus (HBV) have been approved by the Food and Drug Administration. These virus-like particle (VLP) based vaccines elicit strong antibody responses and are highly effective at protecting against HPV and HBV infection.

VLPs can not only be used as stand-alone vaccines, but they can also be utilized as platforms for the multivalent and immunogenic display of heterologous antigens. The family of single-stranded RNA bacteriophage is a versatile platform for displaying these heterologous epitopes. MS2 and PP7 coat protein single-chain dimers are highly tolerant of peptide insertions and produce correctly assembled VLPs displaying the peptide insertion on the surface of VLP in a highly dense, repetitive array. These VLPs are highly immunogenic and confer this high immunogenicity to heterologous peptides displayed on their surfaces.

Current HPV vaccines are based on VLPs comprised of the viral major capsid protein L1. Although LI-VLP vaccines are highly effective, they are type-specific, meaning that the current HPV vaccines only provide protection against a small subset of the more than 100 types of HPVs that infect humans, including the 15-18 HPV types that are carcinogenic. In contrast, the HPV minor capsid protein, L2, contains broadly cross-neutralizing epitopes. Antibodies that are specific for these highly conserved epitopes within L2 are able to neutralize infection by a broad range of HPV types. Therefore, a vaccine targeting L2 might provide more comprehensive protection against infection by multiple HPV types. However, unlike L1, the L2 protein does not assemble into VLPs on its own, and is therefore not very immunogenic, meaning that it is difficult to induce high titer antibodies against it.

Previously we described the use of virus-like particles (VLPs) of two RNA bacteriophages, MS2 and PP7, for peptide display (*see* (10)). As described above, MS2 and PP7 coat protein single-chain dimers are highly tolerant of peptide insertions and produce correctly assembled VLPs displaying the peptide insertion on the surface of VLP in a highly dense, repetitive array. These VLPs are highly immunogenic and confer this high immunogenicity to heterologous peptides displayed on their surfaces. Peptides can be displayed at either of two sites on the bacteriophage capsid protein, in the so-called AB-loop and at the N-terminus.

We have also previously described VLPs displaying a peptide antigens derived from the Human Papillomavirus (HPV) minor capsid protein, L2, in the AB-loop. Such recombinant VLPs could serve as a prophylactic vaccine to prevent infection by diverse HPV strains, and are effective at preventing infection with HPV pseudovirions in animal models. *See e.g.* PCT/US2011/024030, filed February 8, 2011, entitled "Immunogenic HPV L2-Containing VLPs and Related Compositions, Constructs and Therapeutic Methods", published as WO 2011/100234, August 18, 2011, TUMBAN EBENEZER ET AL. PLOS ONE 2011, vol. 6, no. 8, E23310, 2011, disclose a VLP of PP7 single chain dimers with an L2 insert in the AB loop. We describe herein the construction of VLPs displaying L2 peptides at the N-terminus of the bacteriophage coat protein. Surprisingly, these L2-displaying VLPs induce more broadly neutralizing antibody responses than when the same peptide is displayed in the AB-loop. Thus, a single recombinant VLP displaying an L2 peptide can provide broad protection against HPV infection..

### SUMMARY OF THE INVENTION

The invention provides immunotherapeutic and prophylactic bacteriophage viral-like particle (VLPs) which are useful in the prevention of human papillomavirus (HPV) infections and related disorders, including cervical cancer and persistent infections associated with HPV. Related compositions (e.g. vaccines), nucleic acid constructs, and therapeutic methods are also provided. VLPs and related compositions of the invention induce high titer antibody responses against HPV L2 and protect against HPV pseudovirus challenge *in vivo.* VLPs, VLP-containing compositions, of the invention induce an immunogenic response against HPV infection, confer immunity against HPV infection, protect against HPV infection, and reduce the likelihood of infection by HPV infection.

Because antibodies that are specific for highly conserved epitopes within L2 are able to neutralize infection by a broad range of HPV types, HPV L2-targeting VLPs and related compositions (e.g. vaccines) of the invention provide a more comprehensive protection against infection by multiple HPV types.

In one aspect, the invention provides a VLP comprising a bacteriophage single chain coat polypeptide dimer and a HPV L2 peptide, wherein the HPV L2 peptide is displayed on the VLP, and wherein vaccination with the VLP is prophylactic for HPV-induced disorders.

In another aspect, the invention provides a composition comprising a VLP comprising a bacteriophage single chain coat polypeptide dimer and a HPV L2 peptide, wherein the HPV L2 peptide is displayed on the VLP, and wherein the composition is prophylactic for HPV-induced disorders.

More specifically, the invention provides a VLP, or a composition comprising a VLP, wherein the VLP is made by transforming a prokaryote with a nucleic acid construct comprising a bacterial or bacteriophage promoter which is operably associated with a coding sequence of bacteriophage MS2 single chain coat polypeptide dimer, wherein the coat polypeptide dimer coding sequence is modified to (i) define a codon sequence positioned 5' to that portion of the sequence which defines the coat polypeptide dimer N-terminus, and (ii) contain a nucleotide sequence encoding a HPV L2 peptide; (b) a restriction site positioned 3' to the coat polypeptide dimer coding sequence; (c) a PCR primer positioned 3' to the second restriction site; (d) a repressor to resistance to a first antibiotic, wherein the repressor is operably associated with the promoter; (e) a helper phage gene modified to contain a gene conferring resistance to a second antibiotic, and (f) a replication origin for replication in a prokaryotic cell.

In certain aspects, the invention provides VLPs made by transforming a prokaryote with HPV L2 peptide sequence-containing constructs as described herein. In other aspects, VLPs and VLP-containing compositions (e.g. vaccines) of the invention are comprised of VLPs comprising HPV L2 peptides derived from different HPV types. In other aspects, VLPs and VLP-containing compositions of the invention comprise hybrid VLPs that display multiple HPV L2 sequences.

In certain aspects, the invention provides a composition for use in a method of inoculating a subject at risk of developing a HPV-related disorder, the method comprising administering to the subject one or more doses of a composition comprising a HPV L2-containing VLP as described herein.

These and other aspects of the invention are described further in the Detailed Description of the Invention.

### BRIEF DESCRIPTION OF THE FIGURES

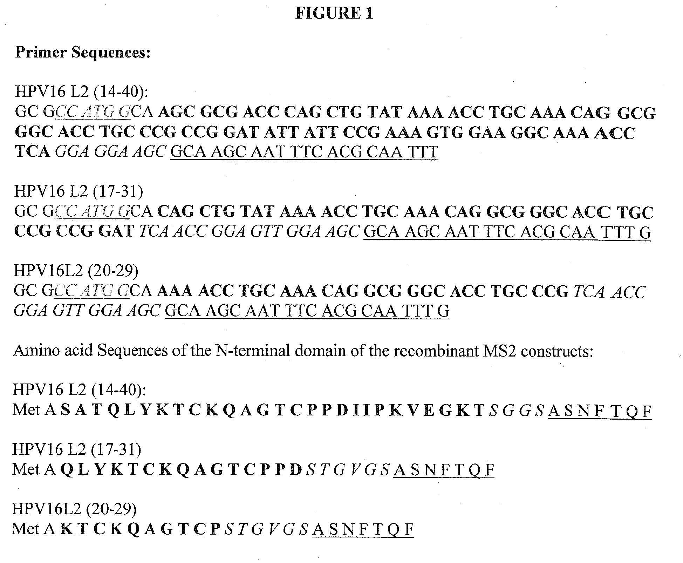

Figure 1 lists the forward primers used to clone the listed sequences into MS2 coat (shown 5' to 3'), as well as the amino acids sequences encoded by these primers. The Ncol restriction site is shown in underlined italics, the peptide insertion is shown in bold text, and the linker sequence is shown in italics. MS2 coat protein sequences are underlined.

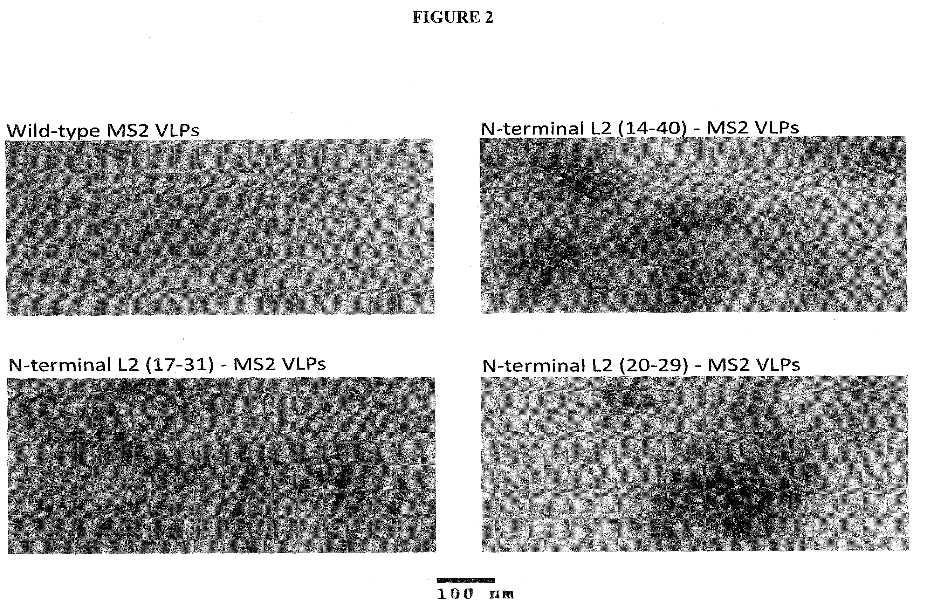

Figure 2 depicts transmission electron micrographs of wild-type and recombinant MS2 VLPs

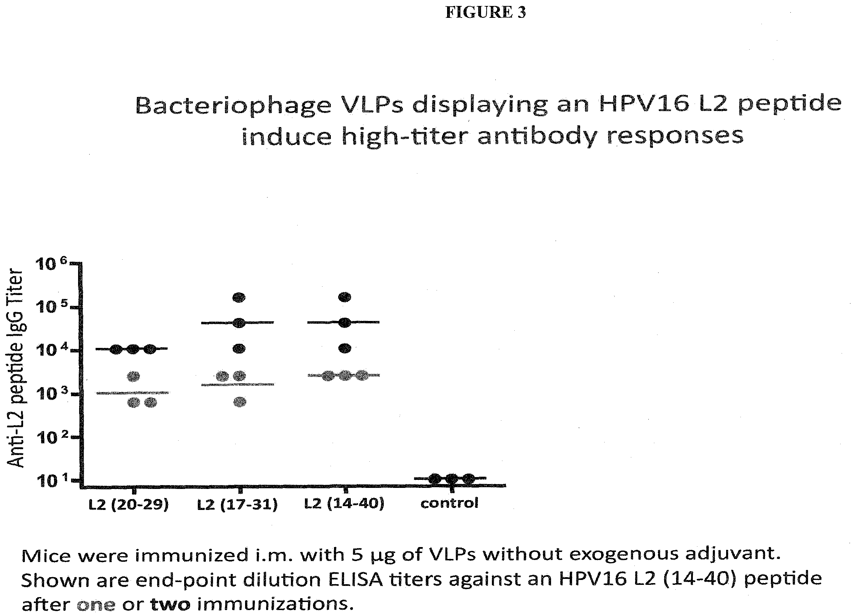

Figure 3. Figure 3 shows the IgG antibody responses in groups of mice immunized with wild-type MS2 VLPs (controls) or MS2 VLPs displaying L2 peptides (20-29), (17-31), or (14-40). Titers were calculated against a peptide representing amino acids 14-40 from HPV16 L2 conjugated to streptavidin. Results are from sera obtained one week after the initial vaccination (red) or one week after the boost (black). Each datum point represents the antibody titer from an individual mouse. Lines represent the geometric mean titer for each group. As indicated, mice were immunized i.m. with 5 µg of VLPs without exogenous adjuvant. Shown are end-point dilution ELISA titers against an HPV16 L2 (14-40) peptide after **one** or **two** immunizations.

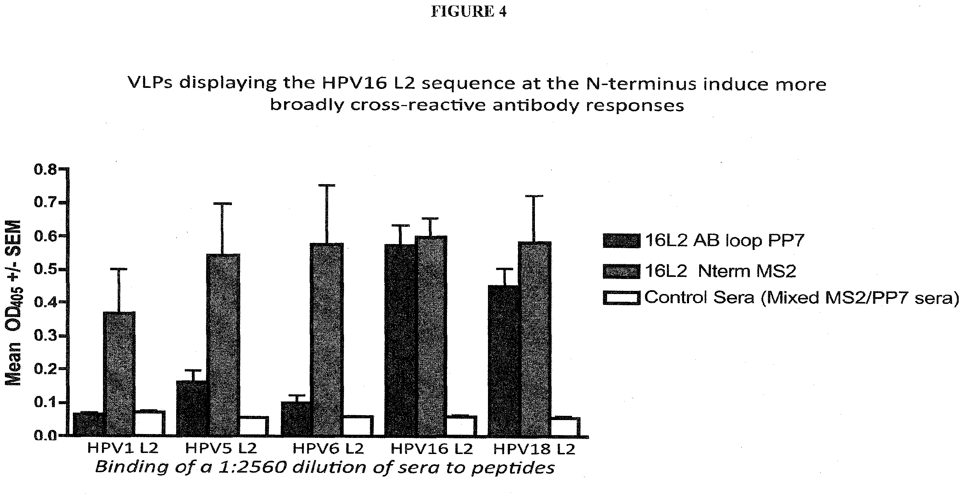

Figure 4. Figure 4 shows the cross-reactivity of sera elicited upon immunization with 16L2(17-31) displayed either at the N-terminus of MS2 coat protein or in the AB-loop of PP7. ELISA plates were coated with the streptavidin-conjugated L2 peptides representing HPV1, 5, 6, 16, & 18 and reacted with a 1:2560 dilution of serum from mice. Shown are the averages of the optical density (OD₄₀₅) values of sera from three individual mice in each group. Error bars represent SEM. As show, VLps displaying the HPV16 L2 sequence at the N-terminus induce more broadly cross-reactive antibody responses.

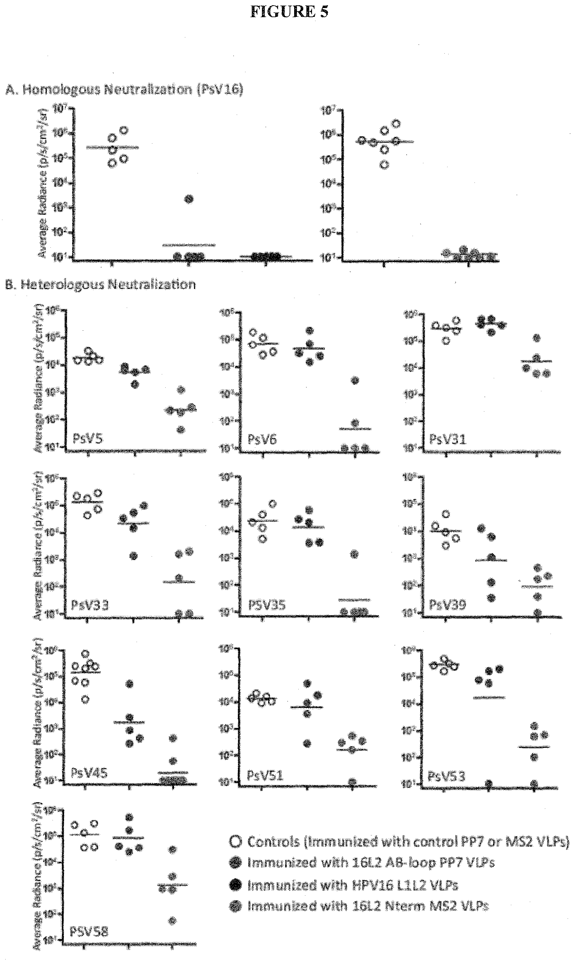

Figure 5. This figure shows that mice immunized with 16L2(17-31) displayed either the N-terminus of MS2 coat protein show broader protection from infection with diverse HPV PsV types compared to those immunized with 16L2(17-31) AB-loop PP7 VLPs. Balb/c mice were immunized (intramuscularly) twice at two weeks intervals with 5 µg of 16L2(17-31) Nterm MS2 VLPs or 16L2(17-31) AB-loop PP7 VLPs or HPV16 L1L2 VLPs or a mixture of MS2/PP7 VLPs. Three to five weeks after the last immunization, mice were challenged with: A) PsV16 and B) PsV5, 6, 31, 33, 35, 39, 45, 51, 53, and PsV58. All challenges were done vaginally except PsV5, which was done intradermally. Forty-eight hours post-challenge, 0.4 mg of luciferin was administered via the same route used for PsV challenge and average radiance (p/s/cm²/sr) values for each mouse was determined using Living Image 3.2 software. White-filled black circles represent mice immunized with MS2/PP7 VLPs, black-filled circles represent mice immunized with HPV16 L1L2 VLPs, blue-filled circles represent mice immunized with 16L2(17-31) AB-loop PP7 VLPs, and red-filled circles represent mice immunized with 16L2(17-31) Nterm MS2 VLPs. Black solid lines represent the geometric mean of average radiance.

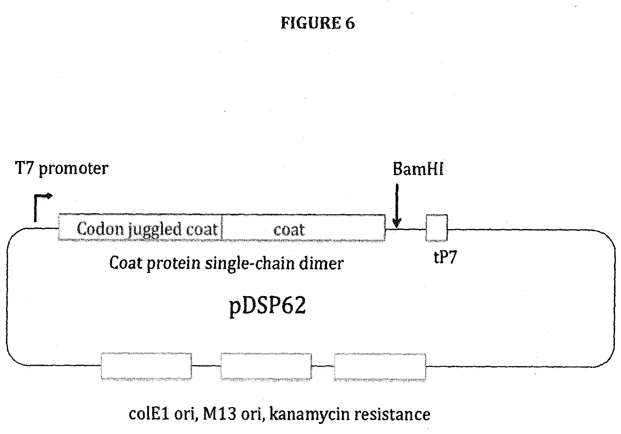

Figure 6 depicts the pDSP62 plasmid.

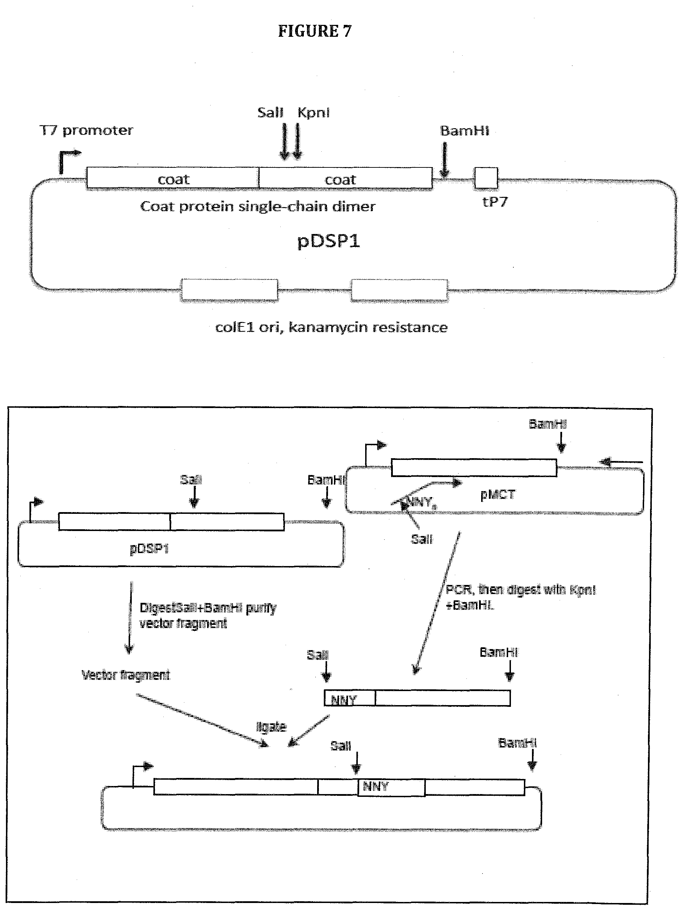

Figure 7 depicts the plasmid pDSP1.

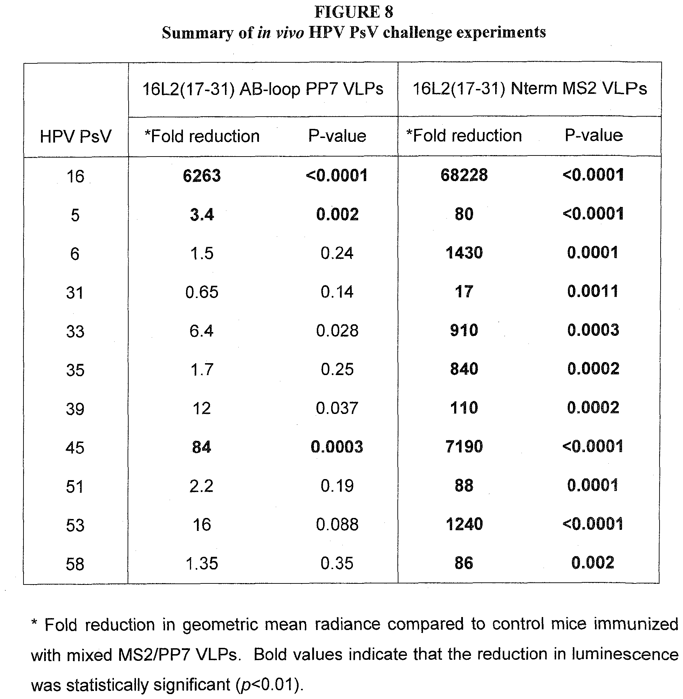

Figure 8 shows that there were dramatic differences in protection from heterologous HPV PsV challenge. Immunization with L2-PP7 VLPs resulted in modest or no protection against heterologous PsV. In contrast, immunization with N-terminal displayed L2-MS2 VLPs according to the present invention resulted in significant protection from vaginal challenge with nine heterologous types and one intradermal challenge with HPV5 PsV. We observed very strong (80- to 7,190-fold reduction in signal) protection from infection with 9 out of the 10 heterologous PsV types tested, an unexpected result. This protection was substantially stronger than what was observed in mice immunized with AB-loop displayed L2. Protection against homologous challenge using VLPs according to the present invention was generally at least about 5-10-fold greater than VLPs where an antigenic L2 peptide was displayed in the AB-loop. Protection against heterologous challenge was 10-25-fold (HPV5, 31 & 39), 40-100-fold (HPV45, 51, 53, & 58), or 140-1000-fold (HPV6, 33, & 35) better using the N-terminal VLP construct.

### DETAILED DESCRIPTION OF THE INVENTION

In accordance with the present invention there may be employed conventional molecular biology, microbiology, and recombinant DNA techniques within the skill of the art. Such techniques are explained fully in the literature. See, e.g., Sambrook et al, 2001, "Molecular Cloning: A Laboratory Manual"; Ausubel, ed., 1994, "Current Protocols in Molecular Biology" Volumes I-III; Celis, ed., 1994, "Cell Biology: A Laboratory Handbook" Volumes I-III; Coligan, ed., 1994, "Current Protocols in Immunology" Volumes I-III; Gait ed., 1984, "Oligonucleotide Synthesis"; Hames & Higgins eds., 1985, "Nucleic Acid Hybridization"; Hames & Higgins, eds., 1984,"Transcription And Translation"; Freshney, ed., 1986, "Animal Cell Culture"; IRL Press, 1986, "Immobilized Cells And Enzymes"; Perbal, 1984, "A Practical Guide To Molecular Cloning."

Where a range of values is provided, it is understood that each intervening value, to the tenth of the unit of the lower limit unless the context clearly dictates otherwise, between the upper and lower limit of that range and any other stated or intervening value in that stated range is encompassed within the invention. The upper and lower limits of these smaller ranges may independently be included in the smaller ranges is also encompassed within the invention, subject to any specifically excluded limit in the stated range. Where the stated range includes one or both of the limits, ranges excluding either both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, the preferred methods and materials are now described.

It must be noted that as used herein and in the appended claims, the singular forms "a," "and" and "the" include plural references unless the context clearly dictates otherwise.

Furthermore, the following terms shall have the definitions set out below.

As used herein, the term "polynucleotide" refers to a polymeric form of nucleotides of any length, either ribonucleotides or deoxynucleotides, and includes both double- and single-stranded DNA and RNA. A polynucleotide may include nucleotide sequences having different functions, such as coding regions, and non-coding regions such as regulatory sequences (e.g., promoters or transcriptional terminators). A polynucleotide can be obtained directly from a natural source, or can be prepared with the aid of recombinant, enzymatic, or chemical techniques. A polynucleotide can be linear or circular in topology. A polynucleotide can be, for example, a portion of a vector, such as an expression or cloning vector, or a fragment.

As used herein, the term "polypeptide" refers broadly to a polymer of two or more amino acids joined together by peptide bonds. The term "polypeptide" also includes molecules which contain more than one polypeptide joined by a disulfide bond, or complexes of polypeptides that are joined together, covalently or noncovalently, as multimers (e g., dimers, tetramers). Thus, the terms peptide, oligopeptide, and protein are all included within the definition of polypeptide and these terms are used interchangeably. It should be understood that these terms do not connote a specific length of a polymer of amino acids, nor are they intended to imply or distinguish whether the polypeptide is produced using recombinant techniques, chemical or enzymatic synthesis, or is naturally occurring.

The amino acid residues described herein are preferred to be in the "L" isomeric form. However, residues in the "D" isomeric form can be substituted for any L-amino acid residue, as long as the desired functional is retained by the polypeptide. NH₂ refers to the free amino group present at the amino terminus of a polypeptide. COOH refers to the free carboxy group present at the carboxy terminus of a polypeptide.

The term "coding sequence" is defined herein as a portion of a nucleic acid sequence which directly specifies the amino acid sequence of its protein product. The boundaries of the coding sequence are generally determined by a ribosome binding site (prokaryotes) or by the ATG start codon (eukaryotes) located just upstream of the open reading frame at the 5'-end of the mRNA and a transcription terminator sequence located just downstream of the open reading frame at the 3'- end of the mRNA. A coding sequence can include, but is not limited to, DNA, cDNA, and recombinant nucleic acid sequences.

A "heterologous" region of a recombinant cell is an identifiable segment of nucleic acid within a larger nucleic acid molecule that is not found in association with the larger molecule in nature.

An "origin of replication" refers to those DNA sequences that participate in DNA synthesis.

A "promoter sequence" is a DNA regulatory region capable of binding RNA polymerase in a cell and initiating transcription of a downstream (3' direction) coding sequence. For purposes of defining the present invention, the promoter sequence is bounded at its 3' terminus by the transcription initiation site and extends upstream (5' direction) to include the minimum number of bases or elements necessary to initiate transcription at levels detectable above background. Within the promoter sequence will be found a transcription initiation, as well as protein binding domains (consensus sequences) responsible for the binding of RNA polymerase. Eukaryotic promoters will often, but not always, contain "TATA" boxes and "CAT" boxes. Prokaryotic promoters contain Shine-Dalgarno sequences in addition to the -10 and -35 consensus sequences.

An "expression control sequence" is a DNA sequence that controls and regulates the transcription and translation of another DNA sequence. A coding sequence is "under the control" of transcriptional and translational control sequences in a cell when RNA polymerase transcribes the coding sequence into mRNA, which is then translated into the protein encoded by the coding sequence. Transcriptional and translational control sequences are DNA regulatory sequences, such as promoters, enhancers, polyadenylation signals, terminators, and the like, that provide for the expression of a coding sequence in a host cell.

A cell has been "transformed" by exogenous or heterologous DNA when such DNA has been introduced inside the cell. The transforming DNA may or may not be integrated (covalently linked) into chromosomal DNA making up the genome of the cell. In prokaryotes, yeast, and mammalian cells for example, the transforming DNA may be maintained on an episomal element such as a plasmid.

It should be appreciated that also within the scope of the present invention are nucleic acid sequences encoding the polypeptide(s) of the present invention, which code for a polypeptide having the same amino acid sequence as the sequences disclosed herein, but which are degenerate to the nucleic acids disclosed herein. By "degenerate to" is meant that a different three-letter codon is used to specify a particular amino acid.

As used herein, "epitope" refers to an antigenic determinant of a polypeptide. An epitope could comprise 3 amino acids in a spatial conformation which is unique to the epitope. Generally an epitope consists of at least 5 such amino acids, and more usually, consists of at least 8-10 such amino acids. Methods of determining the spatial conformation of amino acids are known in the art, and include, for example, x-ray crystallography and 2-dimensional nuclear magnetic resonance.

As used herein, the term "coat protein(s)" refers to the protein(s) of a bacteriophage or a RNA-phage capable of being incorporated within the capsid assembly of the bacteriophage or the RNA-phage.

As used herein, a "coat polypeptide" as defined herein is a polypeptide fragment of the coat protein that possesses coat protein function and additionally encompasses the full length coat protein as well or single-chain variants thereof.

As used herein, the term "immune response" refers to a humoral immune response and/or cellular immune response leading to the activation or proliferation of B- and/or T-lymphocytes and/or antigen presenting cells. In some instances, however, the immune responses may be of low intensity and become detectable only when using at least one substance in accordance with the invention. "Immunogenic" refers to an agent used to stimulate the immune system of a living organism, so that one or more functions of the immune system are increased and directed towards the immunogenic agent. An "immunogenic polypeptide" is a polypeptide that elicits a cellular and/or humoral immune response as described above, whether alone or linked to a carrier in the presence or absence of an adjuvant. Preferably, antigen presenting cell may be activated.

As used herein, the term "vaccine" refers to a formulation which contains the composition of the present invention and which is in a form that is capable of being administered to an animal.

As used herein, the term "virus-like particle of a bacteriophage" refers to a virus-like particle (VLP) resembling the structure of a bacteriophage, being non-replicative and noninfectious, and lacking at least the gene or genes encoding for the replication machinery of the bacteriophage, and typically also lacking the gene or genes encoding the protein or proteins responsible for viral attachment to or entry into the host.

This definition should, however, also encompass virus-like particles of bacteriophages, in which the aforementioned gene or genes are still present but inactive, and, therefore, also leading to non-replicative and noninfectious virus-like particles of a bacteriophage.

VLP of RNA bacteriophage coat protein: The capsid structure formed from the self-assembly of one or more subunits of RNA bacteriophage coat protein and optionally containing host RNA is referred to as a "VLP of RNA bacteriophage coat protein". In a particular embodiment, the capsid structure is formed from the self assembly of 90 coat protein single-chain dimers or 180 coat protein monomers.

A nucleic acid molecule is "operatively linked" to, or "operably associated with", an expression control sequence when the expression control sequence controls and regulates the transcription and translation of nucleic acid sequence. The term "operatively linked" includes having an appropriate start signal (e.g., ATG) in front of the nucleic acid sequence to be expressed and maintaining the correct reading frame to permit expression of the nucleic acid sequence under the control of the expression control sequence and production of the desired product encoded by the nucleic acid sequence. If a gene that one desires to insert into a recombinant DNA molecule does not contain an appropriate start signal, such a start signal can be inserted in front of the gene.

### HPV-Induced Disorders, Immunogenicity, and Prophylactic Efficacy

"HPV-induced disorders" or "HPV-related disorders" include, but are not limited to, the disorders identified in this section. Immunogenicity and prophylactic efficacy (e.g. whether a composition is prophylactic for HPV-induced disorders) may be evaluated either by the techniques and standards mentioned in this section, or through other methodologies that are well-known to those of ordinary skill in the art.

Over 100 different HPV types have been identified and are referred to by number. Types 16, 18, 31, 33, 35, 39, 45, 51, 52, 56, 58, and 59 are a subset of the "high-risk" sexually transmitted HPVs and may lead to the development of cervical intraepithelial neoplasia (CIN), vulvar intraepithelial neoplasia (VIN), vaginal cancer, penile intraepithelial neoplasia (PIN), and/or anal intraepithelial neoplasia (AIN). Several types of HPV, particularly type 16, have been found to be associated with oropharyngeal squamous-cell carcinoma, a form of head and neck cancer. HPV (e.g., HPV 6 and 11) cause genital warts, and HPV 6 and 11 can cause recurrent respiratory papillomatosis. HPV may cause epidermodysplasia verruciformis in immunocompromised individuals.
Thus, high-risk human HPV infection of the cervical epithelium is causally linked with the generation of cervical cancer. See Durst et al., PNAS 80 3812-3815, 1983; Gissmann et al., J. Invest. Dermatol 83 265-285, 1984 (HPV16 is associated with premalignant and malignant diseases of the genito-urinary tract, and in particular with carcinoma of the cervix). Papillomavirus prophylactic vaccines target the systemic immune system for induction of neutralizing antibodies that protect the basal cells against infection.

To assess immunogenicity (e.g. whether a composition has induced a high titer antibody responses against HPV L2), an anti-HPV 16 L2 geometric mean titer (GMT) can be Measured by ELISA, e.g. after a few weeks of treatment (e.g. 3 or 4 weeks) and after administration of a few dosages (e.g. 3 or 4). The percentage of subjects who seroconverted for HPV 16 after a few weeks of treatment (e.g. 3 or 4 weeks) and after administration of a few dosages (e.g. 3 or 4) can also be determined to assess immunogenicity.

To determine prophylactic efficacy, an immunogenicity analysis can be conducted on subjects who remain HPV 16 seronegative and PCR-negative to HPV infection (swab and biopsy) at various endpoints after challenge.

### HPV L2

"HPV L2" as used herein includes the L2 capsid proteins of all human papillomaviruses.

### Production of Virus-Like Particles

The present invention is directed to virus-like phage particles. As used herein, producing virions *"in vitro"* refers to producing virions outside of a cell, for instance, in a cell-free system, while producing virions *"in vivo"* refers to producing virions inside a cell, for instance, an *Escherichia coli* or *Pseudomonas aeruginosa* cell.

### Bacteriophages

The VLPs described here consist of assemblies of the coat proteins of single-strand RNA bacteriophage [RNA Bacteriophages, in The Bacteriophages. Calendar, RL, ed. Oxford University Press. 2005]. The known viruses of this group attack bacteria as diverse as *E. coli, Pseudomonas* and *Acinetobacter.* Each possesses a highly similar genome organization, replication strategy, and virion structure. In particular, the bacteriophages contain a single-stranded (+)-sense RNA genome, contain maturase, coat and replicase genes, and have small (<300 angstrom) icosahedral capsids. These include but are not limited to MS2, PP7, Qb, R17, SP, PP7, GA, M11, MX1, f4, Cb5, Cb12r, Cb23r, 7s and f2 RNA bacteriophages.

The information required for assembly of the icosahedral capsid shell of this family of bacteriophage is contained entirely within coat protein itself. For example, purified coat protein can form capsids *in vitro* in a process stimulated by the presence of RNA [Beckett et al., 1988, J. Mol Biol 204: 939-47]. Moreover, coat protein expressed in cells from a plasmid assembles into a virus-like particle *in vivo* [Peabody, D.S., 1990, J Biol Chem 265: 5684-5689].

Examples of PP7 coat polypeptides include but are not limited to the various chains of PP7 Coat Protein Dimer in Complex With Rna Hairpin (e.g. Genbank Accession Nos. 2QUXR; 2QUXO; 2QUX_L; 2QUX_I; 2QUX_F; and 2QUX_C). *See also* Example 1 herein and Peabody, et al., RNA recognition site of PP7 coat protein, Nucleic Acids Research, 2002, Vol. 30, No. 19 4138-4144.

### RNA Bacteriophage Coat Polypeptide

The coat polypeptides also include those having similarity with one or more of the coat polypeptide sequences disclosed above. The similarity is referred to as structural similarity. Structural similarity may be determined by aligning the residues of the two amino acid sequences (i.e., a candidate amino acid sequence and the amino acid sequence) to optimize the number of identical amino acids along the lengths of their sequences; gaps in either or both sequences are permitted in making the alignment in order to optimize the number of identical amino acids, although the amino acids in each sequence must nonetheless remain in their proper order. A candidate amino acid sequence can be isolated from a single stranded RNA virus, or can be produced using recombinant techniques, or chemically or enzymatically synthesized. Preferably, two amino acid sequences are compared using the BESTFIT algorithm in the GCG package (version 10.2, Madison WI), or the Blastp program of the BLAST 2 search algorithm, as described by Tatusova, et al. (FEMS Microbial Lett 1999, 174:247-250), and available at http://www.ncbi.nlm.nih.gov/blast/bl2seq/bl2.html. Preferably, the default values for all BLAST 2 search parameters are used, including matrix =BLOSUM62; open gap penalty = 11, extension gap penalty = 1, gap xdropoff = 50, expect = 10, wordsize = 3, and optionally, filter on. In the comparison of two amino acid sequences using the BLAST search algorithm, structural similarity is referred to as "identities." Preferably, a coat polypeptide also includes polypeptides with an amino acid sequence having at least 80% amino acid identity, at least 85% amino acid identity, at least 90% amino acid identity, or at least 95% amino acid identity to one or more of the amino acid sequences disclosed above. Preferably, a coat polypeptide is active. Whether a coat polypeptide is active can be determined by evaluating the ability of the polypeptide to form a capsid and package a single stranded RNA molecule. Such an evaluation can be done using an *in vivo* or *in vitro* system, and such methods are known in the art and routine. Alternatively, a polypeptide may be considered to be structurally similar if it has similar three-dimensional structure as the recited coat polypeptide and/or functional activity.

### The HPV L2 peptide

As described herein, the HPV L2 peptide sequence : is present at N-terminus of a coat polypeptide. The HPV L2 peptide sequence is expressed on the outer surface of the capsid in accordance with the examples presented hereinafter.

The HPV L2 peptide sequence corresponds to amino acids 17-31 derived from the minor capsid protein L2 of Human Papillomavirus type 16 (HPV16).

In order to determine a corresponding position in a structurally similar coat polypeptide, the amino acid sequence of this structurally similar coat polypeptide is aligned with the sequence of the named coat polypeptide as specified above. The coat polypeptide is a single-chain dimer containing an upstream and downstream subunit. Each subunit contains a functional coat polypeptide sequence. The HPV L2 peptide sequence is inserted in the downstream subunit at the sites mentioned herein above, i.e., the N-terminus of a downstream subunit. The coat polypeptide is a single chain dimer of a MS2 coat polypeptide.

### Preparation of Transcription Unit

The transcription unit comprises an expression regulatory region, (e.g., a promoter), a sequence encoding a coat polypeptide and transcription terminator. The RNA polynucleotide may optionally include a coat recognition site (also referred to a "packaging signal", "translational operator sequence", "coat recognition site"). Alternatively, the transcription unit may be free of the translational operator sequence. The promoter, coding region, transcription terminator, and, when present, the coat recognition site, are generally operably linked. "Operably linked" or "operably associated with" refer to a juxtaposition wherein the components so described are in a relationship permitting them to function in their intended manner. A regulatory sequence is "operably linked" to, or "operably associated with", a coding region when it is joined in such a way that expression of the coding region is achieved under conditions compatible with the regulatory sequence. The coat recognition site, when present, may be at any location within the RNA polynucleotide provided it functions in the intended manner.

A wide variety of promoters are known. The promoter can be a constitutive or an inducible promoter. Preferred promoters are able to drive high levels of RNA encoded by me coding region encoding the coat polypeptide Examples of such promoters are known in the art and include, for instance, the lac promoter, T7, T3, and SP6 promoters.

The nucleotide sequences of the coding regions encoding coat polypeptides described herein are readily determined. These classes of nucleotide sequences are large but finite, and the nucleotide sequence of each member of the class can be readily determined by one skilled in the art by reference to the standard genetic code. Furthermore, the coding sequence of an RNA bacteriophage single chain coat polypeptide comprises a site for insertion of HPV L2 peptide-encoding sequences. In a particular embodiment, the site for insertion of the HPV L2 peptide-encoding sequence is a restriction enzyme site. In another embodiment, the HPV L2 peptide-encoding sequence is inserted using polymerase chain reaction (PCR) using standard techniques. The coding region encodes a single-chain dimer of the coat polypeptide. The coding region encodes a modified single chain coat polypeptide dimer, where the modification comprises an insertion of a coding sequence at least four amino acids at the insertion site. The transcription unit may contain a bacterial promoter, such as a lac promoter or it may contain a bacteriophage promoter, such as a T7 promoter.

### Synthesis

The VLPs of the present invention may be produced *in vivo* by introducing transcription units into bacteria, especially if transcription units contain a bacterial promoter. Alternatively, it may be synthesized *in vitro* in a coupled cell-free transcription/translation system.

### Assembly of VLPs Encapsidatig Heterologous Substances

As noted above, the VLPs of the present invention encapsidate a HPV L2 peptide-encoding sequence. These VLPs may be also be assembled in combination with another substance, such as an adjuvant. Specifically, purified coat protein subunits are obtained from VLPs that have been disaggregated with a denaturant (usually acetic acid). The adjuvant is mixed with coat protein, which is then reassembled in its presence. In a particular embodiment, the substance has some affinity for the interior of the VLP and is preferably negatively charged.

In another embodiment, the adjuvant is passively diffused into the VLP through pores that naturally exist in the VLP surface. In a particular embodiment, the substance is small enough to pass through these pores and has a high affinity for the interior of the VLP.

### EXAMPLES

The invention may be better understood by reference to the following non-limiting examples, which are provided as exemplary of the invention. References corresponding to numerical reference citations are listed after the examples.

### Bacteriophages PP7 and MS2 and Related Plasmids.

Previously we described the use of virus-like particles of bacteriophage MS2 for peptide display. We established that MS2 coat protein single-chain dimers are highly tolerant of peptide insertions and that they produce correctly assembled VLPs that specifically encapsidate the mRNA encoding their synthesis (Peabody, D. S., Manifold-Wheeler, B., Medford, A., Jordan, S. K., do Carmo Caldeira, J., and Chackerian, B. (2008) J Mol Biol 380, 252-263). As explained above, MS2 is only one member of a large family of viruses whose individual members share similar molecular biology, e.g. PP7, a bacteriophage phage.

### Experimental Overview.

As explained above, we have previously described the use of VLPs of two RNA bacteriophages, MS2 and PP7, for peptide display. MS2 and PP7 coat protein single-chain dimers are highly tolerant of peptide insertions and produce correctly assembled VLPs displaying the peptide insertion on the surface of VLP in a highly dense, repetitive array. These VLPs are highly immunogenic and confer this high immunogenicity to heterologous peptides displayed on their surfaces. Here we also describe VLPs displaying a peptide antigens derived from the Human Papillomavirus (HPV) minor capsid protein, L2. Such recombinant VLPs serve as a prophylactic vaccine to prevent infection by diverse HPV strains. The vaccines described below induced high titer antibody responses against L2 and protected mice against infection with diverse HPV pseudoviruses.

### Example 1

As noted above, current HPV vaccines are based on VLPs comprised of the viral major capsid protein L1. Although L1-VLP vaccines are highly effective, they are type-specific, meaning that the current HPV vaccines only provide protection against a small subset of the more than 100 types of HPVs that infect humans, including the 15-18 HPV types that are carcinogenic. In contrast, the HPV minor capsid protein, L2, contains broadly cross-neutralizing epitopes. Antibodies that are specific for these highly conserved epitopes within L2 are able to neutralize infection by a broad range of HPV types (7). Therefore, a vaccine targeting L2 might provide more comprehensive protection against infection by multiple HPV types. Unlike L1, the L2 protein does not assemble into VLPs on its own, and is therefore not very immunogenic, meaning that it is difficult to induce high titer antibodies against it.

We constructed and established the utility of VLP-based vaccines targeting the HPV L2 protein. The data below describes the construction of recombinant MS2 VLPs that display L2 peptides. These vaccines induce high titer antibody responses against L2 and protect against HPV challenge in a mouse model of infection. Similar techniques could also be used to construct PP7 VLPs that display L2 peptides.

### Design of L2 displaying VLPs:

As noted above, we described previously the construction of the expression plasmid pDSP62 and pDSP1 (3) (Figure 6 and 7). This plasmid codes for the expression of a version of MS2 coat protein in which two copies of coat protein are genetically fused into a "single-chain" dimer. These plasmids contain a unique Ncol restriction site that allows for genetic insertion of sequences at the N-terminus of the single-chain dimer. To create the VLPs that display L2 peptides we designed PCR primers that allowed us to clone three HPV16 L2-derived sequences onto the N-terminus of coat protein. These sequences represented L2 amino acids 20-29, 17-31, and 14-40 from HPV16. The primers used to create these constructs and the amino acid sequences of these insertions are shown in Figure 1.

Modified pDSP62 containing the recombinant sequences was used to express recombinant VLPs in E. *coli.* Following our standard procedures for expression and purification, recombinant VLPs were visualized by transmission electron microscopy. As shown in Figure 2, all three recombinant coat proteins formed VLPs.

### L2 peptides displayed on MS2 VLPs are immunogenic.

To test the immunogenicity of the VLPs, mice were immunized with 16L2-VLPs or wild-type MS2 VLPs by intramuscular injection. Groups of three mice were immunized intramuscularly with 10 µg of VLPs without any exogenous adjuvant. All mice were boosted with the same amount of VLPs two weeks later. Sera were collected one week after each inoculation. Sera from the mice were tested, by end-point dilution ELISA, for IgG antibodies specific for the 16L2 peptide (Figure 3). As shown, mice immunized with all three16L2-VLPs generated high-titer (geometric mean titer > 10⁴) IgG responses against the corresponding peptide whereas no antibodies were detected in control mice. Thus, L2 peptides displayed on the surface of MS2 single-chain dimer VLPs display the high immunogenicity that is characteristic of other VLP-displayed antigens.

### PP7 VLPs displaying a HPV16 L2 peptide can induce neutralizing antibodies that protect mice from homologous and heterologous genital HPV pseudovirus challenge.

The 16L2-VLP vaccine we designed contains amino acids 17-31 from HPV16 L2, a region shown to contain one or more highly cross-reactive neutralizing epitopes (1, 5), suggesting that the 16L2 VLPs could elicit antibodies that broadly cross-react with L2 sequences from other HPV strain and could potentially protect against HPV challenge. First, we assessed whether the sera from mice immunized with recombinant MS2 VLPs displaying HPV16 L2 (17-31) at the N-terminus could cross-react with peptide representing L2 amino acids 14-40 from five HPV strains (HPV1, 5, 6, 16, & 18). As shown in Figure 4, this sera was broadly cross-reactive, reacting with all five peptides. Unexpectedly, sera from mice immunized with PP7 VLPs displaying the same HPV16 L2 peptide in the AB-loop was much less broadly cross-reactive.

To determine whether the broader cross-reactivity observed with the insertion of epitope 17-31 at the N-terminus of MS2 coat protein correlated with protection, mice were immunized with VLPs displaying the HPV16 L2 epitope at either the N-terminus of MS2 coat protein [16L2(17-31) Nterm MS2 VLPs] or at the AB-loop of PP7 coat protein [16L2(17-31) AB-loop PP7 VLPs] or control VLPs, and were then challenged with a panel of HPV pseudoviruses (PsVs) (PsV5, 6, 16, 31, 33, 35, 39, 45, 51, 53, and 58) three to five weeks after the 2^{nd} immunization. As expected, mice immunized with either 16L2(17-31) Nterm MS2 VLPs or 16L2(17-31) AB-loop PP7 VLPs showed almost complete protection from high-dose vaginal challenge with homologous HPV16 PsV (Figure 5A). Protection was similar to mice immunized with HPV16 L1L2 VLPs. However, there were dramatic differences in protection from heterologous HPV PsV challenge (Figure 5B) Immunization with L2-PP7 VLPs resulted in modest or no protection against heterologous PsV. In contrast, immunization with L2-MS2 VLPs resulted in significant protection from vaginal challenge with nine heterologous types and one intradermal challenge with HPV5 PsV. We observed very strong (80- to 7,190-fold reduction in signal) protection from infection with 9 out of the 10 heterologous PsV types tested. Protection from HPV31 PsV was somewhat weaker (17-fold), but still statistically significant (*p*<0.01, one-tailed *t*-test). Thus, immunization with MS2 VLPs displaying a single HPV16 L2 peptide provided protection against diverse HPV pseudovirus types.

Our experiments showed that there were dramatic differences in protection from heterologous HPV PsV challenge. See figure 8. Immunization with L2-PP7 VLPs resulted in modest or no protection against heterologous PsV. In contrast, immunization with N-terminal displayed L2-MS2 VLPs according to the present invention resulted in significant protection from vaginal challenge with nine heterologous types and one intradermal challenge with HPV5 PsV. We observed very strong (about 80- to 7,190-fold reduction in signal) protection from infection with 9 out of the 10 heterologous PsV types tested, an unexpected result. This protection was surprisingly substantially stronger than what was observed in mice immunized with AB-loop displayed L2. Consequently our experimental results evidenced that protection against homologous challenge using VLPs according to the present invention is generally at least about 5-10-fold greater than VLPs where an antigenic L2 peptide is inserted into the AB-loop. In particular, protection against heterologous challenge was shown to be 10-25-fold (HPV5, 31 & 39), 40-100-fold (HPV45, 51, 53, & 58), or 140-1000-fold (HPV6, 33, & 35) better using the N-terminal VLP construct.

### Summary.

Genetic display of peptides on PP7 VLPs is well suited for the precise targeting of specific B-cell epitopes known to be the target of neutralizing antibodies. For many pathogens, including influenza (4, 12), Hepatitis C Virus (8), and HIV (2), the target epitopes of broadly neutralizing antibodies are poorly immunogenic, meaning that full-length proteins are inadequate for the induction of antibody responses by vaccination. On the other hand, the use of peptide epitopes as vaccines is limited because of their poor immunogenicity unless coupled to carrier proteins. The PP7 and MS2 VLP platforms that we have described allow for targeted introduction of specific peptide epitopes in a highly immunogenic context. In this example, we targeted a broadly neutralizing epitope near the N-terminus of the HPV16 L2 protein. This epitope is the target of an HPV neutralizing monoclonal antibody (5), and it was already shown that the corresponding synthetic peptide, when linked to a carrier protein, can elicit cross-neutralizing antibodies against HPV (1). We show that MS2 VLPs displaying the L2 epitope at the N-terminus of the bacteriophage coat protein induce high-titer peptide-specific antibodies, and the antibodies were of high enough titer to provide almost complete protection of mice from genital challenge with homologous (HPV16) as well as heterologous (HPV 5, 6, 31, 33, 35, 39, 45, 51, 53, and 58) pseudovirus. Thus, MS2 VLPs show utility for the targeted induction of antibodies against specific epitopes.

### References:

1. Alphs, H. H., R. Gambhira, B. Karanam, J. N. Roberts, S. Jagu, J. T. Schiller, W. Zeng, D. C. Jackson, and R. B. Roden. 2008. Protection against heterologous human papillomavirus challenge by a synthetic lipopeptide vaccine containing a broadly cross-neutralizing epitope of L2. Proc Natl Acad Sci U S A 105:5850-5.
2. Burton, D. R., R. L. Stanfield, and I. A. Wilson. 2005. Antibody vs. HIV in a clash of evolutionary titans. Proc Natl Acad Sci U S A 102:14943-8.
3. Chackerian, B., C. Caldeira Jdo, J. Peabody, and D. S. Peabody. 2011. Peptide Epitope Identification by Affinity Selection on Bacteriophage MS2 Virus-Like Particles. J Mol Biol 409:225-37.
4. Ekiert, D. C., G. Bhabha, M. A. Elsliger, R. H. Friesen, M. Jongeneelen, M. Throsby, J. Goudsmit, and I. A. Wilson. 2009. Antibody recognition of a highly conserved influenza virus epitope. Science 324:246-51.
5. Gambhira, R., B. Karanam, S. Jagu, J. N. Roberts, C. B. Buck, I. Bossis, H. Alphs, T. Culp, N. D. Christensen, and R. B. Roden. 2007. A protective and broadly cross-neutralizing epitope of human papillomavirus L2. J Virol 81:13927-31.
6. Johnson, K. M., R. C. Kines, J. N. Roberts, D. R. Lowy, J. T. Schiller, and P. M. Day. 2009. Role of heparan sulfate in attachment to and infection of the murine female genital tract by human papillomavirus. J Virol 83:2067-74.
7. Karanam, B., S. Jagu, W. K. Huh, and R. B. Roden. 2009. Developing vaccines against minor capsid antigen L2 to prevent papillomavirus infection. Immunol Cell Biol 87:287-99.
8. Law, M., T. Maruyama, J. Lewis, E. Giang, A. W. Tarr, Z. Stamataki, P. Gastaminza, F. V. Chisari, I. M. Jones, R. I. Fox, J. K. Ball, J. A. McKeating, N. M. Kneteman, and D. R. Burton. 2008. Broadly neutralizing antibodies protect against hepatitis C virus quasispecies challenge. Nat Med 14:25-7.
9. Lim, F., T. P. Downey, and D. S. Peabody. 2001. Translational repression and specific RNA binding by the coat protein of the Pseudomonas phage PP7. J Biol Chem 276:22507-13.
10. Peabody, D. S., B. Manifold-Wheeler, A. Medford, S. K. Jordan, J. do Carmo Caldeira, and B. Chackerian. 2008. Immunogenic display of diverse peptides on Virus-like Particles of RNA phage MS2. Journal of Molecular Biology doi:10.1016/j.jmb/2008.04.049.
11. Roberts, J. N., C. B. Buck, C. D. Thompson, R. Kines, M. Bernardo, P. L. Choyke, D. R. Lowy, and J. T. Schiller. 2007. Genital transmission of HPV in a mouse model is potentiated by nonoxynol-9 and inhibited by carrageenan. Nat Med 13:857-61.
12. Sui, J., W. C. Hwang, S. Perez, G. Wei, D. Aird, L. M. Chen, E. Santelli, B. Stec, G. Cadwell, M. Ali, H. Wan, A. Murakami, A. Yammanuru, T. Han, N. J. Cox, L. A. Bankston, R. O. Donis, R. C. Liddington, and W. A. Marasco. 2009. Structural and functional bases for broad-spectrum neutralization of avian and human influenza A viruses. Nat Struct Mol Biol 16:265-73.

## Claims

1. A composition comprising: a MS2 virus-like particle comprising a MS2 bacteriophage single chain coat polypeptide dimer having at least one antigenic human papillomavirus (HPV) L2 peptide corresponding to amino acids 17-31 of HPV type 16 L2 protein inserted at the amino terminus of the MS2 bacteriophage single chain coat polypeptide; wherein said peptide is displayed on said virus-like particle.

2. The composition of claim 1, wherein said HPV L2 peptide is an HPV type 1, 5, 6, 11, 16, 18, 31, 33, 35, 39, 45, 51, 52, 53, 56, 58, or 59 HPV L2 peptide.

3. The composition of claim 1, wherein the MS2 virus-like particle displays more than one HPV L2 peptide of different HPV types.

4. Composition according to claim 1 for use as a medicament.

5. Composition for use according to claim 4 for use in enhancing an immune response against an HPV in an animal wherein said composition is introduced into said animal, wherein an enhanced immune response against HPV is produced in said animal.

6. Composition for use according to claim 5, wherein said animal is a mammal.

7. Composition for use according to claim 5, wherein said composition 2. is introduced into said animal subcutaneously, intramuscularly, intravenously, intranasally, intravaginally or directly into the lymph node.

8. A vaccine comprising an immunologically effective amount of the composition according to claim 1 together with a pharmaceutically acceptable diluent, carrier or excipient.

9. The vaccine according to claim 8 for use in immunizing or treating a mammal.

10. The vaccine for use according to claim 9, wherein said vaccine is a vaccine for human papillomavirus (HPV).

## Patentansprüche

1. Zusammensetzung, umfassend: einen virusartigen MS2-Partikel, umfassend ein MS2-Bakteriophagen-Einzelkettenhüllpolypeptiddimer mit wenigstens einem antigenen humanen Papillomavirus(HPV)-L2-Peptid, das Aminosäuren 17-31 von HPV-L2-Protein des Typs 16 entspricht und an dem Aminoterminus des MS2-Bakteriophagen-Einzelkettenhüllpolypeptids eingefügt ist; wobei das Peptid an dem virusartigen Partikel angezeigt wird.

2. Zusammensetzung nach Anspruch 1, wobei das HPV-L2-Peptid ein HPV-L2-Peptid des HPV-Typs 1, 5, 6, 11, 16, 18, 31, 33, 35, 39, 45, 51, 52, 53, 56, 58 oder 59 ist.

3. Zusammensetzung nach Anspruch 1, wobei der virusartige MS2-Partikel mehr als ein HPV-L2-Peptid unterschiedlicher HPV-Typen anzeigt.

4. Zusammensetzung nach Anspruch 1 zur Verwendung als ein Medikament.

5. Zusammensetzung zur Verwendung nach Anspruch 4 zur Verwendung beim Stärken einer Immunantwort auf ein HPV in einem Tier, wobei die Zusammensetzung in das Tier eingebracht wird, wobei eine verstärkte Immunantwort auf HPV in dem Tier erzeugt wird.

6. Zusammensetzung zur Verwendung nach Anspruch 5, wobei das Tier ein Säuger ist.

7. Zusammensetzung zur Verwendung nach Anspruch 5, wobei die Zusammensetzung subkutan, intramuskulär, intravenös, intranasal, intravaginal oder direkt in den Lymphknoten in das Tier eingebracht wird.

8. Impfstoff, umfassend eine immunologisch wirksame Menge der Zusammensetzung nach Anspruch 1 zusammen mit einem pharmazeutisch akzeptablen Verdünnungsmittel, Träger oder Hilfsstoff.

9. Impfstoff nach Anspruch 8 zur Verwendung beim Immunisieren oder Behandeln eines Säugers.

10. Impfstoff zur Verwendung nach Anspruch 9, wobei der Impfstoff ein Impfstoff für humanes Papillomavirus (HPV) ist.

## Revendications

1. Une composition comprenant : une particule de type viral MS2 comprenant un dimère polypeptidique d'enveloppe monocaténaire du bactériophage MS2 ayant au moins un peptide L2 antigénique du virus du papillome humain (HPV) correspondant aux acides aminés 17-31 de la protéine L2 du HPV de type 16 insérée au niveau du N-terminal du polypeptide d'enveloppe monocaténaire du bactériophage MS2 ; où ledit peptide est présent sur ladite particule de type viral.

2. La composition selon la revendication 1, dans laquelle ledit peptide HPV L2 est un peptide HPV L2 de type HPV 1, 5, 6, 11, 16, 18, 31, 33, 35, 39, 45, 51, 52, 53, 56, 58, ou 59.

3. La composition selon la revendication 1, dans laquelle la particule de type viral MS2 présente plus d'un peptide HPV L2 de types HPV différents.

4. La composition selon la revendication 1, destinée à être utilisée comme un médicament.

5. La composition à utiliser selon la revendication 4, destinée à être utilisée pour améliorer la réponse immunitaire contre un HPV chez un animal, où ladite composition est administrée chez ledit animal, où une réponse immunitaire améliorée contre le HPV est produite chez ledit animal.

6. La composition à utiliser selon la revendication 5, où ledit animal est un mammifère.

7. La composition à utiliser selon la revendication 5, où ladite composition est administrée chez ledit animal par voie sous-cutanée, intramusculaire, intraveineuse, intranasale, intravaginale ou directement dans le noeud lymphoïde.

8. Un vaccin comprenant une quantité immunologiquement effective de la composition selon la revendication 1 avec un diluant, vecteur ou excipient pharmaceutiquement acceptable.

9. Le vaccin selon la revendication 8, destiné à être utilisé pour immuniser ou traiter un mammifère.

10. Le vaccin à utiliser selon la revendication 9, où ledit vaccin est un vaccin pour le virus du papillome humain (HPV).
